# EUROPEAN PATENT SPECIFICATION

(11) **EP 3 131 593 B1**
(45) Date of publication and mention of the grant of the patent: **14.10.2020**
(21) Application number: 15716799.0
(22) Date of filing: 16.04.2015
(51) Int. Cl.: A61L 27/54

(54) **MEANS AND METHODS FOR PREVENTING OR TREATING DENTAL IMPLANT ASSOCIATED INFLAMMATION**
MITTEL UND VERFAHREN ZUR VORBEUGUNG ODER BEHANDLUNG VON ENTZÜNDUNGEN IM ZUSAMMENHANG MIT ZAHNIMPLANTATEN
MOYENS ET PROCÉDÉS DE PRÉVENTION OU DE TRAITEMENT D'INFLAMMATION ASSOCIÉE À UN IMPLANT DENTAIRE

(30) Priority: 17.04.2014 EP 14165087
(43) Date of publication of application: 22.02.2017
(73) Proprietor: Actimplant AG, CH-6300 Zug (CH)
(72) Inventor: KAMBER, Michael, 4411 Seltisberg (CH)
(74) Representative: Altmann Stößel Dick Patentanwälte PartG mbB
(86) International application number: PCT/EP2015/058268
(87) International publication number: WO 2015/158825

(56) References cited:
- WO-A1-2008/045928
- WO-A1-2011/132096
- MARTINEZ ET AL.: "Bone loss at implant with Titanium abutments coated by soda lime glass containing silver nanoparticles: a histological stuy in the dog N", PLOS ONE, vol. 9, no. 1, January 2014 (2014-01), XP002730768,
- ROBERTO LOPEZ-PIRIZ: "Radiolic Evaluation of bone loss at implants with biocide coated titanium abutments: a study in the dog", PLOS ONE, vol. 7, no. 12, 2012, pages 1-6, XP002730769,

## Description

The present disclosure relates to a dental implant or an element thereof comprising a trans-mucosal portion, wherein at least a part of said trans-mucosal portion comprises a biocide. The present disclosure further relates to the use of a biocide for manufacturing a dental implant or element thereof, and to a biocide released from a dental implant or an element thereof according to the present invention for use in preventing and/or treating dental implant associated inflammation. Further the present disclosure relates to the use of a biocide for manufacturing a dental implant or element thereof.

Any references in the description to methods of treatment refer to the biocide for use in a method for treatment of the human body by therapy. Dental implants are an important option in oral rehabilitation. Typically, dental implants comprise a bone-integral part, also known as enossal or osseous part, anchoring the implant in the jawbone of the patient and a trans-mucosal portion spanning the gingiva/mucosa. Said parts of the implant may be combined in one single implant (one-piece or tissue-level implant), or may be embodied in two or more parts; in the case of two- or more piece implants (bone-level implant), typically the osseous part is separate from other elements, whereas the trans-mucosal portion and the crown may be separate parts or may be combined. In any case, the replacement of the visible part of the tooth, the crown, will be fixed onto the trans-mucosal portion. It is an advantage of two- or more-piece implants that the trans-mucosal portion may be changed while leaving the enossal portion in place. Thus, in two- or more-piece embodiments, the trans-mucosal element may be embodied as a mucosal former or as a healing cap, also termed "healing abutment", when used during healing, or as an abutment, also known as "secondary element" for anchoring the crown in fixed restorations, or as "retention element" for fixing the prosthesis in removable restorations.

A common problem of dental implants is the formation of a, mostly bacterial, biofilm between the implant and the mucosa. This biofilm is not accessible to mechanical removal with a toothbrush, and frequently is the cause of inflammatory processes leading to peri-implant mucositis, which generally is reversible after appropriate treatment, or to peri-implantitis (Berglundh et al, (2011), J Clin Periodontol 38 Suppl 11: 188). Peri-implant mucositis has been estimated to occur in 80% of patients carrying dental implants, and at 50% of dental implants installed. What is more, peri-implantitis is diagnosed in 28 to 50% of dental implant patients and at 12 to 43% of dental implants installed (Zitzmann and Berglund (2008), J Clin Periodontol 35: 286). Untreated peri-implantitis can lead to bone defects and to loss of the implant.

For this reason, several methods of controlling biofilm formation on dental implants have been proposed, with an emphasis on mechanical removal of biofilms. Other attempts aimed at destroying biofilms by electric currents (Sahrmann et al. (2012), Clin Implant Dent Relat Res, doi: 10.1111/cid.12018, epub ahead of print). Recently, it was proposed to introduce grooves into the trans-mucosal portion of dental implants in order to improve contact between the mucosa and the trans-mucosal implant portion, in an attempt to exclude biofilms from said area (Nevins et al. (2012), Int J Periodontics Restorative Dent 32: e131). In a similar train of thought, it was attempted to cover the trans-mucosal portion of an implant with adhesion molecules, e.g. laminin; however, it was found that such adhesion molecules are degraded over time, making such treatment ineffective (Länge et al. (2004), Dent Mater 20: 814-822). Further, implant abutments coated with soda-lime glass containing silver nanoparticles were experimentally used in a peri-implantitis model, but showed increased surface roughness and cracking of the coating (Lopez-Piriz et al. 2012, PLOS One 7(12), e86926, Martinez et al. 2014, PLOS One 9(1), e52861). Moreover, usability of such coatings in treatment of humans appeared questionable due to the black color of the coatings. Further, WO2011 /132096 teaches an implanting device whose surface combines antibacterial activity so as to prevent periprosthetic infections and improved osseointegration capacity, wherein at least one part of the surface of the device is coated with a hyaluronic acid complex having a glycopeptide antibiotic.

There is, thus, a need in the art for improved means and methods for preventing biofilm formation on dental implants, in particular on the trans-mucosal portion thereof. Such improved means and methods are provided by the embodiments of the present invention.

Accordingly, the present invention relates to a dental implant or an element thereof according to claim 1: Further disclosure relates to a dental implant or an element thereof comprising a trans-mucosal portion, wherein at least a part of said trans-mucosal portion comprises a biocide. The trans-mucosal portion can also be an integral part of the implant crown restoration.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention, and without any restriction regarding the possibility of combining the features introduced in such way with other optional or non-optional features of the invention.

The term "dental implant" is understood by the skilled person and relates to an element anchoring the restoration (crown of an artificial tooth or prosthesis) in a jawbone of a subject. As detailed herein above, dental implants may be manufactured as one-piece embodiments, as two-piece embodiments, or as three or more-piece embodiments. A one-piece dental implant, preferably, comprises at least (i) an enossal portion, anchoring the dental implant in the bone and (ii) a trans-mucosal portion spanning the gum/ mucosa. It will be understood by the skilled person that the final dental implant, preferably, further comprises (iii) a crown, replacing the visible part of the tooth in the oral cavity. In two- or more-piece embodiments, preferably, at least one of the aforesaid elements is embodied as a discrete element. More preferably, in two- or more-piece embodiments, the enossal portion is embodied as a discrete element, the enossal element. Most preferably, in two- or more-piece embodiments, at least the enossal portion and the trans-mucosal portion are embodied as discrete elements. Preferably, in such a case, the enossal portion is a discrete element comprising a female screw thread, into which at least a trans-mucosal element can be anchored, preferably via a screw connection. Preferably, in two- or more-piece embodiments, the trans-mucosal element is a mucosal former, a healing cap (healing abutment), an abutment (secondary element) for fixed restorations, or a retention element for removable restorations. The abutment, preferably, can also be integrated into the final crown restoration, so that the crown is directly connected to the osseous implant part. Accordingly, the term "dental implant or element thereof comprising a trans-mucosal portion", preferably, relates to a one-piece dental implant comprising a trans-mucosal portion; or the term relates to an element of a two- or more-piece dental implant comprising a trans-mucosal portion, i.e., preferably, a mucosal former, a healing cap, an abutment, a retention element or a crown as specified herein above. In a preferred embodiment, the dental implant is a long-term implant, i.e. preferably, a permanent implant; preferably, said long-term implant is implanted for at least one year, more preferably for at least five years, still more preferably for at least ten years, most preferably, for at least 20 years.

The term "trans-mucosal portion", as used herein, relates to a portion of a dental implant or part thereof at least partially spanning the mucosa covering the jawbone, i.e. spanning the gum/ mucosa. Accordingly, preferably, the trans-mucosal portion of a dental implant or element thereof is a portion spanning the section between the jawbone and the oral cavity. It is understood by the skilled person that, preferably, the trans-mucosal portion extends up to 2 mm, more preferably 1 mm into the oral cavity. Most preferably, however, the trans-mucosal portion is even with the mucosa or ends just below the mucosal margin, i.e. does not extend into the oral cavity.

The term "microorganism", as used herein, is understood by the skilled person and relates to any unicellular, multicellular, or viral organism, which is invisible to the naked eye. Preferably, the microorganism is a eukaryotic or prokaryotic organism. More preferably, the microorganism is a bacterium or a yeast, most preferably of a species which is part of the oral flora. Preferably, the bacterium according to the present invention is a member of one of the genera Streptococcus, Fusobacterium, and Actinomyces. More preferably, the bacterium is Streptococcus viridens, S. mitis, S. oralis, S. mutans, or S. sobrinus. Most preferably, the bacterium is Porphyromonas gingivalis or P. intermedia. Preferably, the yeast according to the present invention is a member of the genus Candida, more preferably the yeast is C. albicans.

As used herein, the term "biocide" relates to any compound killing or inhibiting proliferation of at least one microorganism species and/or mediating immunosuppression. Preferably, the biocide is an antibacterial, antifungal, and/or antiviral compound. Preferably, the biocide is compatible with use in the oral cavity. The term "compatible with use in the oral cavity", preferably, relates to the property of a biocide of not inducing severe adverse effects when administered in an effective concentration in the oral cavity. More preferably, compatible with use in the oral cavity relates to the property of a biocide of not inducing moderate adverse effects when administered in an effective concentration in the oral cavity. Most preferably, compatible with use in the oral cavity relates to the property of a biocide of not inducing adverse effects when administered in an effective concentration in the oral cavity. Preferred biocides of the present invention are compounds having antibacterial and/or immunosuppressive properties. As will be understood by the skilled person, the biocide, preferably, is released from the trans-mucosal portion of the dental implant or an element thereof according to the present invention, i.e., preferably, the biocide diffuses from said trans-mucosal portion of the dental implant or an element thereof into the vicinity, preferably into the layer between the mucosa and the trans-mucosal portion of the dental implant or an element thereof.

Preferably, the biocide is a preservative. A preferred preservative according to the present invention is benzoic acid or a derivative thereof, an antioxidant, sorbic acid or a derivative thereof, a propionate salt, a nitrite salt, a sulfite salt, or ethylenediaminetetraacetic acid (EDTA).

Preferably, the biocide is an antimycotic or an antiseptic compound, e.g. chlorhexidine. Preferred antimycotics of the present invention are clotrimazole, nystatin, fluconazole, and ketoconazole.

Also preferably, the biocide comprises or is an antibacterial compound. Antibacterial compounds, preferably, are bactericidal compounds, bacteriostatic compounds, or compounds inhibiting bacterial adhesion to a dental implant, more preferably of bacterial adhesion to the trans-mucosal portion. Preferred bactericidal compounds are antibiotics, including, preferably, at least one of the antibiotics amoxicillin, amoxicillin in combination with clavulanic acid, metronidazole, clindamycin, azithromycin, ciprofloxacin, tetracyclines (e.g. doxycycline, minocycline), or any arbitrary combination thereof (van Winkelhoff, A. J. (2012) European journal of oral implantology Suppl 5; S43-50). Preferred bacteriostatic compounds are bacteriostatic antibiotics including, e.g. preferably, sulfonamides, macrolides, and chloramphenicols. Preferred compounds inhibiting bacterial adhesion are surfactants.

Preferably, the biocide comprises or consists of at least one metal showing an oligodynamic effect, preferably, silver (Ag), gold (Au), mercury (Hg), copper (Cu), iron (Fe), lead (Pb), zinc (Zn), bismuth (Bi), platinum (Pt), cerium (Ce), and aluminum (Al). Preferably, the biocide comprises at least one of the aforesaid at least one metal in the form of an organic complex, e.g., preferably, as allantoinate, alginate, or as a complex with carboxymethylcellulose. More preferably, the biocide comprises at least one of the aforesaid at least one metal as ions, e.g. in the form of an oxide, a phosphate, carbonate, chloride, or sulfate, or in the form of a mixed salt, e.g. Ag/Ca/Na-Phosphate and the like. The skilled person knows how to select organic complexes or salts with appropriate properties, e.g., preferably, appropriate solubility and compatibility with the material of the dental implant. Still more preferably, the biocide comprises at least one of the aforesaid at least one metal in the metallic form, preferably as a solid or finely dispersed, e.g. as nanoparticles. Most preferably, the biocide comprises an alloy comprising at least one of the aforesaid metals. More preferably, the biocide is an alloy comprising at least one of Ag, Au, and Hg. Most preferably, the biocide is an amalgam comprising Hg and Ag or/and Au. In a preferred embodiment, the biocide is not silver nanoparticles comprised in a soda lime glass.

Immunosuppressive compounds are known to the skilled person. Preferably, the immunosuppressive compound is a glucocorticoid, an alkylating agent, an antimetabolite, an immunophilin interacting agent, or a mycophenolate. Preferably, the glucocorticoid is cortisol or dexamethasone. Preferably, the alkylating agent is a nitrogen mustard, e.g. cyclophosphamide, or a platinum compound. The antimetabolite, preferably, is selected from the list consisting of folic acid inhibitors or nucleotide analogues. Preferred immunophilin interacting agents are cyclosporine, tacromlimus, and pimecrolimus.

The biocide, preferably, is not a compound mediating adhesion of mucosal cells, such as laminin, fibronectin or nanoporous TiO₂, and is not a growth factor acting on cells of the mucosa. More preferably, the biocide is not a polypeptide; most preferably, the biocide is not a biological macromolecule. In a preferred embodiment, the biocide does not comprise hyaluronic acid. In a further preferred embodiment, the biocide does not comprise a hyaluronic acid linked to a glycopeptide antibiotic, in particular vancomycin.

Preferably, the release of the biocide from the dental implant or element thereof is essentially constant over time, "essentially constant" relating to a decrease or increase of release of the biocide from the dental implant or element thereof, preferably of not more than 50%, more preferably of not more than 30%, most preferably of not more than 20% over a given time period. It will be understood by the skilled person that the dental implant or element thereof may be used for long-term decrease of biofilm formation on a dental implant. In such an application, the release of biocide from the dental implant or element thereof, preferably is essentially constant over at least six months, more preferably over at least two years, most preferably over at least five years. Further, the dental implant or element thereof may be used for sanitization of an existing implant site. In such an application, the release of biocide from the dental implant or element thereof, preferably is essentially constant over at least one week, more preferably over at least two weeks, most preferably over at least five weeks. It is understood by the skilled person that the term release, preferably, relates to a release of the biocide at such a rate that an effective concentration is obtained, preferably obtained in the cleft between the trans-mucosal portion and the mucosa. In a preferred embodiment, the release of biocide from the dental implant or element thereof is used for prevention or treatment of inflammation.

Preferably, the biocide is comprised in an appropriate carrier. A preferred carrier comprises the biocide of the present invention and is adapted to release said biocide at a constant, preferably slow, rate. Preferred carriers are gels or plastics, more preferably with a pore size appropriate for adequate release of the biocide. Appropriate carriers with the aforesaid properties are known to the skilled person.

Preferably, the biocide is comprised as an integral constituent within said trans-mucosal portion. Thus, the biocide, preferably is included in the material of the dental implant or element thereof in a manner allowing diffusion of the biocide into the surrounding medium. E.g., in case the biocide is an alloy or an amalgam, said alloy or amalgam may be the material the trans-mucosal portion consists of. Alternatively, the biocide, preferably the alloy or amalgam, may be permanently fixed to the trans-mucosal element as a ring or as one or more pins, e.g. by compression molding or by an other method known to the skilled person. In a preferred embodiment, the biocide is permanently fixed to the dental implant or element thereof as a ring fitting in a groove of said trans-mucosal element. Also preferably, the biocide is comprised in a removable means, preferably a ring, mountable onto the trans-mucosal portion. It is however also envisaged that the biocide is comprised in a screw fitting into one or more female screw thread(s) present in the trans-mucosal portion. As will be appreciated by the skilled person, the use of removable means is particularly advantageous in sanitization of existing implant sites, since the kind of biocide comprised in the removable means may be adjusted according to needs. E.g., preferably, in a first step, a bacterial infection may be resolved by administering an antimicrobial compound as specified elsewhere herein, and in a second step, inflammation may be healed by administering an immunosuppressant. In a third step, preferably, recurrent infection may be prevented by providing an implant releasing a biocide according to the present invention. Also preferably, the biocide is comprised in a cavity connected to the environment via at least one aperture comprised in said trans-mucosal portion. More preferably, the biocide is comprised in said cavity as a slowly-dissolving solid or within a carrier, as specified herein above. Thus, in a preferred embodiment, the biocide is comprised in a container abutment. In a further preferred embodiment, said biocide is comprised in a removable container abutment, which can be specifically used as a dispenser for a biocide, preferably in a sanitizing phase.

The dental implant or element thereof of the present invention applies the trans-mucosal portion of said device as a dispenser, inhibiting biofilm formation on said trans-mucosal portion. Since dental implant associated inflammation events are associated with growth of microorganisms in said portion of the dental implant, such inflammation can be ameliorated or prevented.

The definitions made above apply mutatis mutandis to the following. Additional definitions and explanations made further below also apply for all embodiments described in this specification mutatis mutandis.

The present disclosure also relates to a method for preventing biofilm formation on a trans-mucosal portion of a dental implant after implantation, comprising providing a dental implant or an element thereof according to the present invention, and thereby preventing biofilm formation on a dental implant after implantation.

The present disclosure also relates to a method for preventing biofilm formation on a dental prosthesis comprising at least one dental tooth and a denture base or an element thereof, said method comprising providing a dental prosthesis comprising a biocide, and, thereby, preventing biofilm formation on a dental prosthesis.

The method for preventing biofilm formation, preferably, is an in vivo method. Moreover, it may comprise steps in addition to those explicitly mentioned above. Preferably, said method is performed on a healthy individual. More preferably, said method is a cosmetic method administered to avoid the enossal/ osseous implant portion to become visible in the oral cavity. Exposure of the implant surface would be encountered with color deviation and cosmetic impairment.

The term "preventing biofilm formation" refers to impeding or precluding biofilm formation for a certain period of time. It will be understood that said period of time is dependent on the amount of the biocide which is released, and on individual factors of the subject carrying the dental implant or element thereof. It is to be understood that prevention may not be effective in all subjects carrying the dental implant or element thereof according to the present invention. However, the term requires that a statistically significant portion of subjects of a cohort or population are effectively prevented from biofilm formation. Preferably, a cohort or population of subjects is envisaged in this context, which normally, i.e. without preventive measures according to the present invention, would develop a biofilm as referred to herein. Whether a portion is statistically significant can be determined without further ado by the person skilled in the art using various well known statistic evaluation tools discussed elsewhere in this specification.

The term "biofilm" is known to the skilled person and relates to a group of microorganisms in which cells adhere to each other on a surface. Preferably, bacterial cells are the cells mediating adherence to a dental implant in the biofilm according to the present invention. Also preferably, the biofilm is a biofilm located between a dental implant and the surrounding mucosa and/or around the mucosal margin.

Also preferably, the biofilm is a biofilm located at the denture teeth and/or, more preferably, between the denture base and the mucosa. Disorders associated with biofilm formation between the denture base and the mucosa are, e.g., preferably, denture stomatitis, chronic atrophic candidiasis, Candida-associated denture induced stomatitis, and denture-associated erythematous stomatitis.

The present disclosure further relates to a method for preventing or treating dental implant associated inflammation, comprising providing a dental implant or an element thereof according to the present invention, implanting said dental implant or an element thereof at a site afflicted or suspected to become afflicted with dental implant associated inflammation, thereby preventing or treating dental implant associated inflammation.

The method for preventing or treating dental implant associated inflammation, preferably, is an in vivo method. Moreover, it may comprise steps in addition to those explicitly mentioned above.

The term "preventing", as used in the context of the method for preventing or treating dental implant associated inflammation refers to retaining health with respect to the diseases or disorders referred to herein for a certain period of time in a subject. It is to be understood that prevention may not be effective in all subjects treated with the compound according to the present invention. However, the term requires that a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification.

The term "treating" refers to ameliorating the diseases or disorders referred to herein or the symptoms accompanied therewith to a significant extent. Said treating as used herein also includes an entire restoration of the health with respect to the diseases or disorders referred to herein, preferably resolution of dental implant associated inflammation. It is to be understood that treating as used in accordance with the present invention may not be effective in all subjects to be treated. However, the term shall require that a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 60%, at least 70%, at least 80%, or at least 90% of the subjects of a given cohort or population.

As used herein, the term "dental implant associated inflammation" relates to inflammatory processes occurring at the site in an oral cavity where a dental implant was implanted. Preferably, said inflammation is located in a region of the peri-implant mucosa contacting a dental implant. More preferably, dental implant associated inflammation is dental peri-implant mucositis and/or dental peri-implantitis.

Moreover, the present invention also relates to a biocide for use according to claim 8. Further disclosure relates to a biocide released from a dental implant or from an element thereof according to the present invention for use in preventing and/or treating dental implant associated inflammation.

In a preferred embodiment, at least two biocides are administered for preventing and/or, preferably, treating dental implant associated inflammation. In a preferred embodiment, said least two biocides are administered simultaneously. In a more preferred embodiment, said least two biocides are administered one after the other, i.e., successively.

Further, the present invention relates to a method for producing a dental implant or an element thereof according to claim 12. Further disclosure relates to a method for producing a dental implant or an element thereof, comprising including a biocide in at least a part of a trans-mucosal portion of said dental implant or element thereof.

Moreover, the present invention relates to a use of a biocide according to claim 13. Further disclosure relates to a use of a biocide for manufacturing a dental implant or element thereof, wherein, preferably, the dental implant is a dental implant or element thereof according to the present invention.

### Figure Legends

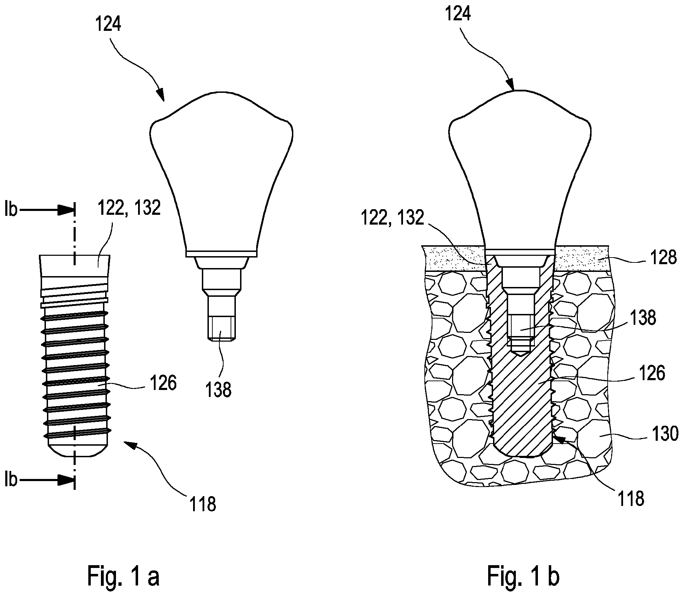

- Fig. 1: shows a schematic representation of a one-piece (tissue-level) dental implant 118 and a restoration (crown) 124. The enossal portion 126 is fixed in the bone 130 and comprises the trans-mucosal portion 122 spanning the mucosa 128 and comprising the biocide 132. The restoration (crown) 124 is fixed into the dental implant 118 via screw thread 138; a) side view, b) cross-sectional view of in situ situation along plane Ib - Ib.

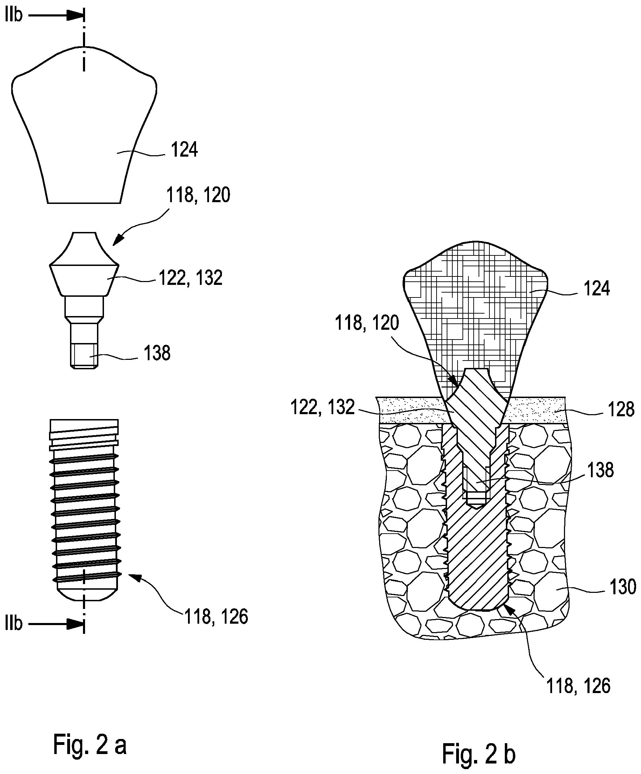

- Fig. 2: shows a schematic representation of a two-piece (bone-level) dental implant 118 with an enossal element 118, 126 and a distal, trans-mucosal element 118, 120. Further shown is a restoration (crown) 124. The enossal portion 126 is fixed in the bone 130. The distal, trans-mucosal element (abutment) 118, 120 with transmucosal portion 122 is connected to the enossal element 118, 126 via a screw thread 138; the restoration (crown) 124 is plugged onto the distal, trans-mucosal element 118, 120; the transmucosal portion 122 spans the mucosa 128 and comprises the biocide 132; a) side view, b) cross-sectional view of in situ situation along plane IIb - IIb.

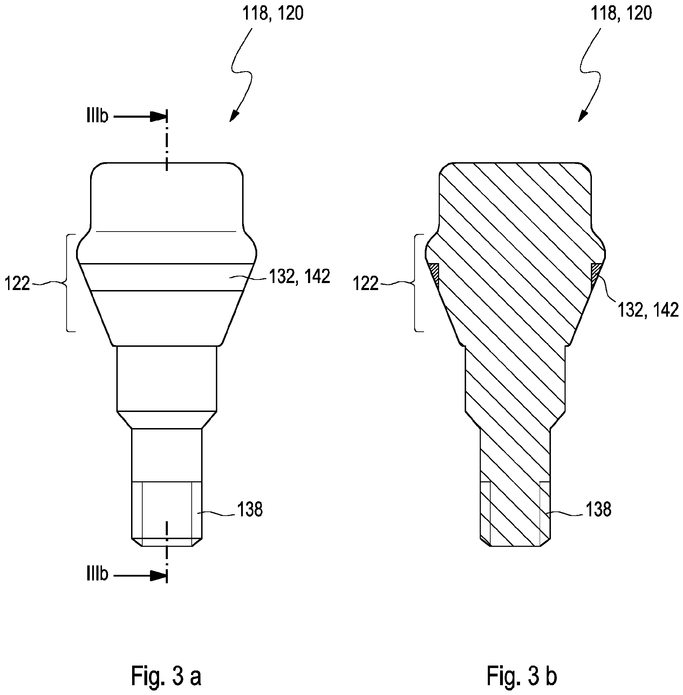

- Fig. 3: shows a schematic representation of a distal, trans-mucosal element 118, 120 comprising a trans-mucosal portion 122, a screw thread 138 for connecting the element to an enossal element, and a, preferably removable, ring 142 comprising the biocide 132 and which, preferably, fits into a corresponding groove in the trans-mucosal element 118, 120; a) side view b) cross-sectional view along plane IIIb - IIIb.

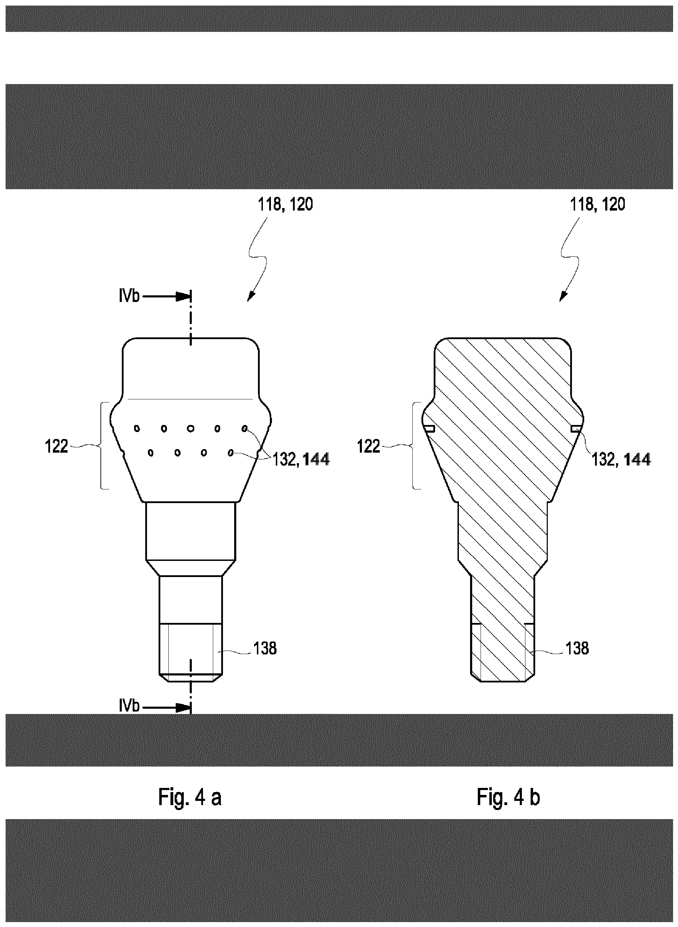

- Fig. 4: shows a schematic representation of a distal, trans-mucosal element 118, 120 comprising a trans-mucosal portion 122, a screw thread 138 for connecting the element to an enossal element, and an array of pins 144 comprising the biocide 132; a) side view b) cross-sectional view along plane IVb - IVb.

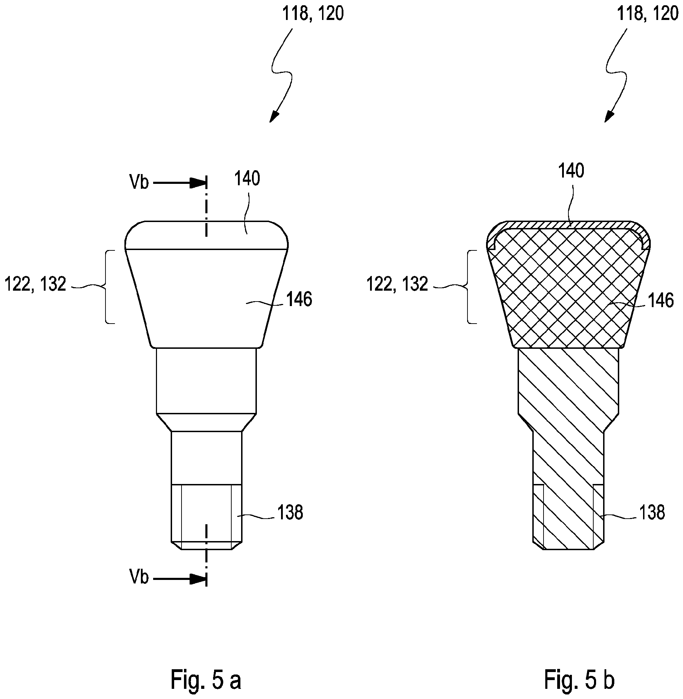

- Fig. 5: shows a schematic representation of a distal, trans-mucosal element 118, 120 having a screw thread 138 for connecting the element to an enossal element. The abutment further comprises a polymeric portion 146 with a trans-mucosal portion 122 comprising the biocide 132. The element can be equipped with a covering plate 140; a) side view b) cross-sectional view along plane Vb - Vb.

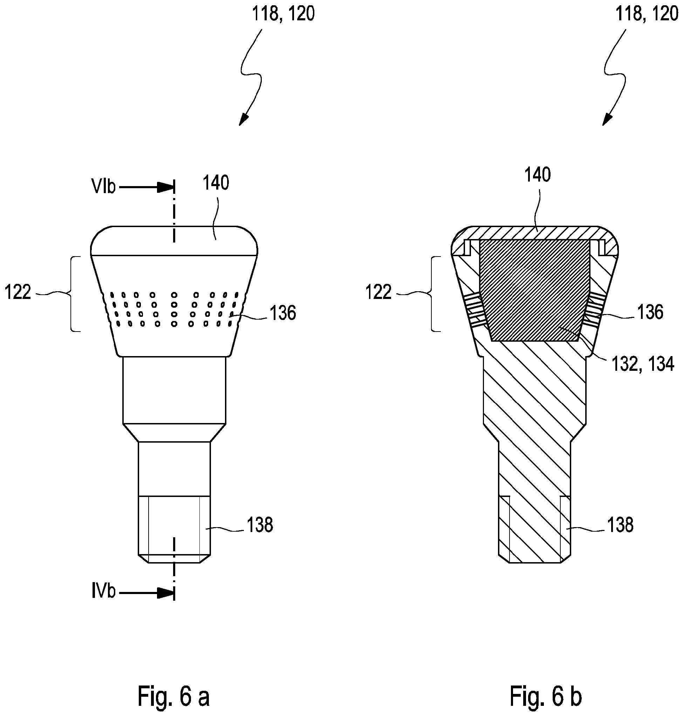

- Fig. 6: shows a schematic representation of a distal, trans-mucosal element 118, 120 comprising a trans-mucosal portion 122 and a screw thread 138 for connecting the element to an enossal portion. The abutment 118, 120 further comprises a cavity 134 comprising the biocide 132 and an array of apertures 136; a) side view b) cross-sectional view along plane VIb - VIb.

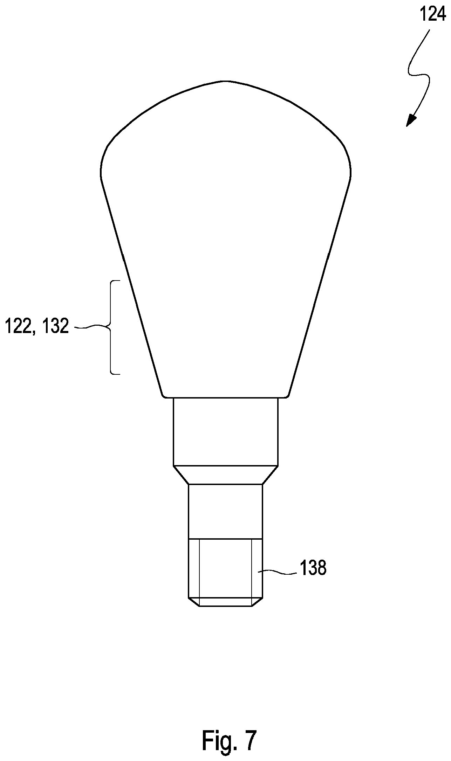

- Fig. 7: shows a schematic representation of an implant restoration (crown) 124 having a screw thread 138. A trans-mucosal portion 122 of the implant restoration (crown) 124 comprises the biocide 132.

The following Examples shall merely illustrate the invention. They shall not be construed, whatsoever, to limit the scope of the invention.

### Examples

In a preferred embodiment, a complete dental implant 118 with restoration (crown) 124 of the present invention is a device as schematically represented in Fig. 1. The device comprises a one-piece (tissue level) dental implant 118 comprising an enossal portion 126 and a trans-mucosal portion 122, which contains the biocide 132. It is understood by the skilled person that the one-piece dental implant 118 will be covered with a restoration (crown) 124 to form a complete tooth replacement. Preferably, only the portion of the one-piece implant 118 protruding into the oral cavity will be covered with a restoration (crown) 124. Preferably, the trans-mucosal portion 122 may consist of the biocide, e.g. an appropriate metal or alloy thereof. The enossal portion 126 of the aforesaid embodiment will heal into the bone 130 of the patient; thus, this embodiment will be particularly suited as a permanent implant, wherein the biocide 132 preferably has the function to prevent biofilm formation and/or dental implant associated inflammation from initiating.

The following embodiments include descriptions of distal elements of two- or more-piece dental implants, which can, preferably, be removed while leaving the enossal element in place. Accordingly, these embodiments are suited for the same purpose as the one-piece embodiment described before. However, the following embodiments are also particularly suited for treating prevalent dental implant associated inflammation. In the following embodiments, preferably, the distal, trans-mucosal element 118, 120 may be a mucosal former, a healing cap (healing abutment), an abutment (secondary element) for fixed restorations, or a retention element for removable restorations. In preferred embodiments, the polymeric abutment according to Fig. 5 and the container abutment comprising a cavity according to Fig. 6 are used for temporary treatment of prevalent dental implant associated inflammation, whereas the embodiments according to Fig. 3 and 4 are preferably used in long-term prevention of biofilm formation and/or inflammation, in particular peri-implantitis.

In a preferred embodiment, the dental implant 118 of the present invention is an element as schematically represented in Fig. 2. In this embodiment, the trans-mucosal portion 122 is part of a distal element 118, 120 of a two-piece dental implant 118, which is connected to the osseous, proximal implant element 126 by a screw thread 138. It is understood by the skilled person that the distal element 118, 120 of the dental implant 118 may be covered with a crown 124 to complete the tooth replacement. Preferably, only the portion of the dental implant 118 protruding into the oral cavity will be covered with a crown 124. Preferably, the trans-mucosal portion 122 may consist of the biocide, e.g. an appropriate metal or alloy thereof. It is also envisaged that the distal, trans-mucosal element 118, 120 may be replaced during treatment in order to adjust the amount of biocide 132 released and/or to adjust the kind of biocide 132 released to the healing process. E.g. in the treatment of prevalent dental implant associated inflammation (preferably, dental peri-implant mucositis and/or dental peri-implantitis), a distal, trans-mucosal element 118, 120 releasing high concentrations of bactericidal and/or fungicidal compounds may be used initially, which may at a later stage be replaced, at least in part, by a distal, trans-mucosal element 118, 120 releasing, e.g., immunosuppressive compounds.

In a preferred embodiment, the distal, trans-mucosal element 118, 120 of the present invention is an element (abutment) as schematically represented in Fig. 3. The element is a distal part 120 of a two-piece dental implant 118 and comprises a trans-mucosal portion 122 and a screw thread 138 for connecting the element to an enossal element 126. The element further comprises a, preferably removable, ring 142 comprising the biocide 132. The ring 142 may comprise or consist of the biocide 132, e.g. preferably, the ring 142 may be composed of a plastic or other polymeric material of sufficient durability comprising the biocide 132; or the ring 142 may comprise or consist of a metal of the present invention and/or an alloy or amalgam thereof. If necessary, the skilled person knows how to design the element of the dental implant on order to reduce abrasive forces, lysis or other deleterious effects onto the ring 142. In a more preferred embodiment, said ring 142 is a metal ring comprising at least one of the metals of the present invention as biocide 132, preferably in an effective concentration. In case the ring 142 shall be removable, the ring 142, preferably, consists of a flexible material to enable removal or exchange of the ring 142; or the ring 142 is a snap ring. It is also envisaged that a ring 142 may be replaced during treatment in order to adjust the amount of biocide 132 released and/or to adjust the kind of biocide 132 released to the healing process. E.g. in the treatment of prevalent dental implant associated inflammation (preferably, dental peri-implant mucositis and/or dental peri-implantitis), a ring 142 releasing high concentrations of bactericidal and/or fungicidal compounds may be used initially, which may at a later stage be replaced, at least in part, by a ring 142 releasing, e.g., immunosuppressive compounds. The ring 142 fits into a groove comprised in said trans-mucosal element. Preferably, the ring 142 is molded into said groove and is therefore in a stable state and facilitates biocide release over years; the latter embodiment is particularly preferred for the preventive aspects and to decrease biofilm adherence.

In another preferred embodiment, the distal, trans-mucosal element 118, 120 of the present invention is an element as schematically represented in Fig. 4. The distal, trans-mucosal element 118, 120 is similar to the element of the previous embodiment. However, the biocide is comprised in pins 144 inserted into the trans-mucosal portion 122 of the element 118, 120. It is understood by the skilled person that the pins 144 may also be replaced by screws comprising a biocide 132. It is also envisaged by the present invention that more than one type of pin or screw 144 is inserted, each of said types of pin or screw 144 comprising a biocide different from the biocide comprised in the other type(s) of pin or screw 144. Moreover, it is also envisaged that at least one pin or screw 144 may be replaced during treatment in order to adjust the amount of biocide 132 released and/or to adjust the kind of biocide 132 released to the healing process. E.g. in the treatment of prevalent dental implant associated inflammation (preferably, dental peri-implant mucositis and/or dental peri-implantitis), pins or screws 144 releasing high concentrations of bactericidal and/or fungicidal compounds may be used initially, which may at a later stage be replaced, at least in part, by pins or screws 144 releasing, e.g., immunosuppressive compounds. In a preferred embodiment, the pins or screws 144 comprise or consist of a metal, an alloy, or amalgam of the present invention acting as biocide. Preferably, the biocide of the present invention is molded into pin-shaped apertures and, preferably, in a stable state for release over years; the latter embodiment is particularly preferred for the preventive aspects and to decrease biofilm adherence.

In another preferred embodiment, the distal, trans-mucosal element 118, 120 of the present invention is an element as schematically represented in Fig. 5. The distal, trans-mucosal element 118, 120 is similar to the element as represented in Fig. 2. However, the biocide 132 is comprised in polymeric, preferably acrylic resin, portion 146 comprising the biocide 132. Preferably, in case the polymeric portion 146 consists of a material of low durability, preferably in use for inflammation treatment, said polymeric portion 146 is covered by covering plate 140, e.g. to withstand mechanical forces acting on said element. It will be understood by the skilled person that the covering plate 140 will preferably consist of a material of higher durability compared to the material the polymeric portion 146; e.g. preferably, the covering plate 140 consists of metal.

In a further preferred embodiment, the distal, trans-mucosal element 118, 120 of the present invention is an element as schematically represented in Fig. 6. The distal, trans-mucosal element 118, 120 is a distal part of a two-piece dental implant 118 and comprises a trans-mucosal portion 122 and a screw thread 138 for connecting the element to an enossal portion 126. The element further comprises a cavity 134 comprising the biocide 132, preferably in admixture with an appropriate carrier, and at least one aperture 136 to allow diffusion of the biocide 132 into the cleft between the mucosa 128 and the distal, trans-mucosal element 118, 120. As will be understood by the skilled person, the covering plate 140 may be fixed or removable and, in case the covering plate is removable, the biocide 132 comprised in the cavity 134 may be changed during treatment. It is, however, also envisaged that the covering plate is contiguous with the body of the distal, trans-mucosal element 118, 120. Alternatively, the covering plate may be omitted from the distal, trans-mucosal element 118, 120 and be included in a restoration (crown). In a preferred embodiment, the container abutment with the cavity harboring the biocide is used for inflammation treatment.

In another preferred embodiment, the element of a dental implant 118 of the present invention is a restoration (crown) 124 as schematically represented in Fig. 7, having a screw thread 138 for connecting the element to an enossal portion 126. The restoration 124, comprises the trans-mucosal portion 122 which comprises the biocide 132. It will be understood that this is a preferred embodiment in which the restoration 124 extends at least partially into the mucosa of the jawbone.

### List of reference numbers

- 118: dental implant
- 120: distal, trans-mucosal element
- 122: trans-mucosal portion
- 124: restoration (crown)
- 126: enossal portion or element
- 128: mucosa (gingiva)
- 130: bone
- 132: biocide or biocide comprising means
- 134: cavity
- 136: aperture
- 138: screw thread
- 140: covering plate
- 142: ring
- 144: pin or screw
- 146: polymeric portion

## Claims

1. A dental implant (118) or an element thereof comprising a trans-mucosal portion (122), wherein at least a part of said trans-mucosal portion (122) comprises a biocide (132), wherein said biocide is a compound killing or inhibiting proliferation of at least one microorganism species and/or is a compound mediating immunosuppression, and wherein said biocide (132)
(i) is comprised as an integral constituent within said trans-mucosal portion (122);
(ii) is permanently fixed to the trans-mucosal portion (122) as a ring (142) fitting into a groove in said trans-mucosal portion (122), or as one or more pins (144) in the trans-mucosal portion (122),
(iii) is comprised in a cavity (134) of said dental implant (118) or element thereof, wherein said cavity (134) is connected to the environment via at least one aperture (136) comprised in said trans-mucosal portion (122); or
(iv) is comprised in a removable means mountable onto said trans-mucosal portion (122).

2. The dental implant (118) or element thereof of claim 1, wherein said biocide (132) is comprised in a removable means, wherein said removable means is a ring (142) or one or more pins (144) mountable onto said trans-mucosal portion (122).

3. The dental implant (118) or element thereof of claim 1 or 2, wherein said compound mediating immunosuppression is an anti-inflammatory compound, preferably an immunosuppressive compound, and wherein the biocide is preferably an antibacterial, antimycotic, antiviral and/or antiseptic.

4. The dental implant (118) or element thereof of claim 3, wherein said antibacterial compound is a bactericidal compound, a bacteriostatic compound, or a compound inhibiting bacterial adhesion to a dental implant.

5. The dental implant (118) or element thereof of claim 4, wherein said bactericidal compound is a metal, preferably at least one of silver (Ag), mercury (Hg) and gold (Au), or an antibiotic, preferably amoxicillin, amoxicillin in combination with clavulanic acid, metronidazole, clindamycin, azithromycin, ciprofloxacin, a tetracycline, or any arbitrary combination thereof.

6. The dental implant (118) or element thereof of claim 3, wherein said immunosuppressive compound is a glucocorticoid, an alkylating agent, an antimetabolite, an immunophilin interacting agent, or a mycophenolate.

7. The dental implant (118) or element thereof of any one of claims 1 to 6, wherein, after implantation, said biocide is released from said part of said trans-mucosal portion at an essentially constant rate for at least one week, preferably for at least four weeks, more preferably for at least six months, most preferably for at least two years.

8. A biocide (132) for use in preventing and/or treating dental implant associated inflammation, wherein said biocide (132) is released from a dental implant (118) according to any one of claims 1 to 7.

9. The biocide (132) for use according to claim 8, wherein said dental implant associated inflammation is dental peri-implant mucositis and/or dental peri-implantitis.

10. The biocide (132) for use according to claim 8 or 9, wherein said use is treating dental implant associated inflammation and wherein said treating comprises administering at least two different biocides, preferably successively administering at least two different biocides.

11. The biocide (132) for use according to any one of claims 8 to 10, wherein said use is treating dental implant associated inflammation and wherein said treating comprises administering an antimicrobial compound followed by administration of an immunosuppressant.

12. A method for producing a dental implant (118) or an element thereof, comprising including a biocide in at least an element of a trans-mucosal portion (122) of said dental implant (118) or element thereof, wherein said biocide is a compound killing or inhibiting proliferation of at least one microorganism species and/or mediating immunosuppression; and wherein said biocide (132)
(i) is included as an integral constituent within said trans-mucosal portion (122);
(ii) is permanently fixed to the trans-mucosal portion (122) as a ring (142) fitting into a groove in said trans-mucosal portion (122), or as one or more pins (144) in the trans-mucosal portion (122), or
(iii) is included in a cavity (134) of said dental implant (118) or element thereof, wherein said cavity (134) is connected to the environment via at least one aperture (136) comprised in said trans-mucosal portion (122); or
(iv) is included in a removable means mountable onto said trans-mucosal portion (122).

13. Use of a biocide for manufacturing a dental implant (118) or element thereof, wherein said dental implant (118) is a dental implant according to any one of claims 1 to 7.

## Patentansprüche

1. Zahnimplantat (118) oder Element davon, umfassend einen transmukosalen Teil (122), wobei wenigstens ein Teil des transmukosalen Teils (122) ein Biozid (132) umfasst, wobei das Biozid eine Verbindung ist, die wenigstens eine Mikroorganismusspezies abtötet oder ihre Proliferation hemmt, und/oder eine Verbindung ist, die Immunsuppression vermittelt, und wobei das Biozid (132)
(i) als integraler Bestandteil in dem transmukosalen Teil (122) enthalten ist;
(ii) als Ring (142), der in eine Rille in dem transmukosalen Teil (122) passt, oder als ein oder mehrere Stifte (144) in dem transmukosalen Teil (122) dauerhaft an dem transmukosalen Teil (122) befestigt ist;
(iii) in einem Hohlraum (134) des Zahnimplantats (118) oder Elements davon enthalten ist, wobei der Hohlraum (134) durch wenigstens eine in dem transmukosalen Teil (122) enthaltene Öffnung (136) mit der Umgebung verbunden ist; oder
(iv) in einer entfernbaren Vorrichtung enthalten ist, die an dem transmukosalen Teil (122) anbringbar ist.

2. Zahnimplantat (118) oder Element davon gemäß Anspruch 1, wobei das Biozid (132) in einer entfernbaren Vorrichtung enthalten ist, wobei die entfernbare Vorrichtung ein Ring (142) oder ein oder mehrere Stifte (144) ist, der/die an dem transmukosalen Teil (122) anbringbar ist/sind.

3. Zahnimplantat (118) oder Element davon gemäß Anspruch 1 oder 2, wobei die Verbindung, die Immunsuppression vermittelt, eine entzündungshemmende Verbindung ist, vorzugsweise eine immunsuppressive Verbindung, und wobei das Biozid vorzugsweise ein antibakterielles Mittel, antimykotisches Mittel, antivirales Mittel und/oder antiseptisches Mittel ist.

4. Zahnimplantat (118) oder Element davon gemäß Anspruch 3, wobei die antibakterielle Verbindung eine bakterizide Verbindung, eine bakteriostatische Verbindung oder eine Verbindung, die Bakterienanhaftung an ein Zahnimplantat verhindert, ist.

5. Zahnimplantat (118) oder Element davon gemäß Anspruch 4, wobei die bakterizide Verbindung ein Metall ist, vorzugsweise wenigstens eines von Silber (Ag), Quecksilber (Hg) und Gold (Au), oder ein Antibiotikum, vorzugsweise Amoxicillin, Amoxicillin in Kombination mit Clavulansäure, Metronidazol, Clindamycin, Azithromycin, Ciprofloxacin, ein Tetracyclin oder eine beliebige Kombination davon.

6. Zahnimplantat (118) oder Element davon gemäß Anspruch 3, wobei die immunsuppressive Verbindung ein Glucocorticoid, ein Alkylierungsmittel, ein Antimetabolit, ein Immunophilin-wechselwirkendes Mittel oder ein Mykophenolat ist.

7. Zahnimplantat (118) oder Element davon gemäß einem der Ansprüche 1 bis 6, wobei nach Implantation das Biozid von dem Teil des transmukosalen Teils mit einer im Wesentlichen konstanten Rate für wenigstens eine Woche, vorzugsweise für wenigstens vier Wochen, bevorzugter für wenigstens sechs Monate, höchst bevorzugt für wenigstens zwei Jahre, freigesetzt wird.

8. Biozid (132) zur Verwendung zur Vorbeugung und/oder Behandlung von Zahnimplantat-verbundener Entzündung, wobei das Biozid (132) aus einem Zahnimplantat (118) gemäß einem der Ansprüche 1 bis 7 freigesetzt wird.

9. Biozid (132) zur Verwendung gemäß Anspruch 8, wobei die Zahnimplantat-verbundene Entzündung dentale Periimplantat-Mucositis und/oder dentale Periimplantitis ist.

10. Biozid (132) zur Verwendung gemäß Anspruch 8 oder 9, wobei die Verwendung Behandlung von Zahnimplantat-verbundener Entzündung ist und wobei die Behandlung Verabreichung von wenigstens zwei verschiedenen Bioziden, vorzugsweise aufeinanderfolgende Verabreichung von wenigstens zwei verschiedenen Bioziden, umfasst.

11. Biozid (132) zur Verwendung gemäß einem der Ansprüche 8 bis 10, wobei die Verwendung Behandlung von Zahnimplantat-verbundener Entzündung ist und wobei die Behandlung Verabreichung einer antimikrobiellen Verbindung, gefolgt von Verabreichung eines immunsuppressiven Mittels, umfasst.

12. Verfahren zur Herstellung eines Zahnimplantats (118) oder eines Elements davon, umfassend Einschließen eines Biozids in wenigstens ein Element eines transmukosalen Teils (122) des Zahnimplantats (118) oder Elements davon, wobei das Biozid eine Verbindung ist, die wenigstens eine Mikroorganismusspezies abtötet oder ihre Proliferation hemmt und/oder Immunsuppression vermittelt; und wobei das Biozid (132)
(i) als integraler Bestandteil in dem transmukosalen Teil (122) enthalten ist;
(ii) als Ring (142), der in eine Rille in dem transmukosalen Teil (122) passt, oder als ein oder mehrere Stifte (144) in dem transmukosalen Teil (122) dauerhaft an dem transmukosalen Teil (122) befestigt ist; oder
(iii) in einem Hohlraum (134) des Zahnimplantats (118) oder Elements davon enthalten ist, wobei der Hohlraum (134) durch wenigstens eine in dem transmukosalen Teil (122) enthaltene Öffnung (136) mit der Umgebung verbunden ist; oder
(iv) in einer entfernbaren Vorrichtung enthalten ist, die an dem transmukosalen Teil (122) anbringbar ist.

13. Verwendung eines Biozids zur Herstellung eines Zahnimplantats (188) oder Elements davon, wobei das Zahnimplantat (118) ein Zahnimplantat gemäß einem der Ansprüche 1 bis 7 ist.

## Revendications

1. Implant dentaire (118) ou élément de celui-ci comprenant une partie transmuqueuse (122), dans lequel au moins une partie de ladite partie transmuqueuse (122) comprend un biocide (132), dans lequel ledit biocide est un composé tuant ou inhibant la prolifération d'au moins une espèce de microorganisme et/ou est un composé médiant l'immunosuppression, et dans lequel ledit biocide (132)
(i) est inclus en tant que constituant intégral dans ladite partie transmuqueuse (122) ;
(ii) est fixé de façon permanente à la partie transmuqueuse (122) sous la forme d'un anneau (142) s'ajustant dans une rainure dans ladite partie transmuqueuse (122), ou sous la forme d'une ou plusieurs broches (144) dans la partie transmuqueuse (122),
(iii) est inclus dans une cavité (134) dudit implant dentaire (118) ou élément de celui-ci, dans lequel ladite cavité (134) est reliée à l'environnement par l'intermédiaire d'au moins une ouverture (136) comprise dans ladite partie transmuqueuse (122) ; ou
(iv) est inclus dans un moyen amovible pouvant être monté sur ladite partie transmuqueuse (122).

2. Implant dentaire (118) ou élément de celui-ci selon la revendication 1, dans lequel ledit biocide (132) est compris dans un moyen amovible, dans lequel ledit moyen amovible est un anneau (142) ou une ou plusieurs broches (144) pouvant être montés sur ladite partie transmuqueuse (122) .

3. Implant dentaire (118) ou élément de celui-ci selon la revendication 1 ou 2, dans lequel ledit composé médiant une immunosuppression est un composé anti-inflammatoire, de préférence un composé immunosuppresseur, et dans lequel le biocide est de préférence un antibactérien, un antimycotique, un antiviral et/ou un antiseptique.

4. Implant dentaire (118) ou élément de celui-ci selon la revendication 3, dans lequel ledit composé antibactérien est un composé bactéricide, un composé bactériostatique ou un composé inhibant l'adhésion bactérienne à un implant dentaire.

5. Implant dentaire (118) ou élément de celui-ci selon la revendication 4, dans lequel ledit composé bactéricide est un métal, de préférence au moins l'un parmi l'argent (Ag), le mercure (Hg) et l'or (Au), ou un antibiotique, de préférence l'amoxicilline, l'amoxicilline en association avec l'acide clavulanique, le métronidazole, la clindamycine, l'azithromycine, la ciprofloxacine, une tétracycline, ou une combinaison arbitraire de ceux-ci.

6. Implant dentaire (118) ou élément de celui-ci selon la revendication 3, dans lequel ledit composé immunosuppresseur est un glucocorticoïde, un agent d'alkylation, un antimétabolite, un agent d'interaction avec les immunophilines, ou un mycophénolate.

7. Implant dentaire (118) ou élément de celui-ci selon l'une quelconque des revendications 1 à 6, dans lequel, après implantation, ledit biocide est libéré depuis ladite partie de ladite partie transmuqueuse à un taux pratiquement constant pendant au moins une semaine, de préférence pendant au moins quatre semaines, plus préférablement pendant au moins six mois, de manière préférée entre toutes pendant au moins deux ans.

8. Biocide (132) pour utilisation dans la prévention et/ou le traitement d'une inflammation associée à un implant dentaire, ledit biocide (132) étant libéré depuis un implant dentaire (118) selon l'une quelconque des revendications 1 à 7.

9. Biocide (132) pour utilisation selon la revendication 8, ladite inflammation associée à un implant dentaire étant une mucosité péri-implantaire dentaire et/ou une péri-implantite dentaire.

10. Biocide (132) pour utilisation selon la revendication 8 ou 9, ladite utilisation étant le traitement d'une inflammation associée à un implant dentaire et ledit traitement comprenant l'administration d'au moins deux biocides différents, de préférence l'administration successive d'au moins deux biocides différents.

11. Biocide (132) pour utilisation selon l'une quelconque des revendications 8 à 10, ladite utilisation étant le traitement d'une inflammation associée à un implant dentaire et ledit traitement comprenant l'administration d'un composé antimicrobien suivie de l'administration d'un immunosuppresseur.

12. Procédé de production d'un implant dentaire (118) ou d'un élément de celui-ci, comprenant un biocide dans au moins un élément d'une partie transmuqueuse (122) dudit implant dentaire (118) ou élément de celui-ci, dans lequel ledit biocide est un composé tuant ou inhibant la prolifération d'au moins une espèce de microorganisme et/ou médiant l'immunosuppression ; et dans lequel ledit biocide (132)
(i) est inclus en tant que constituant intégral dans ladite partie transmuqueuse (122) ;
(ii) est fixé de façon permanente à la partie transmuqueuse (122) sous la forme d'un anneau (142) s'ajustant dans une rainure dans ladite partie transmuqueuse (122), ou sous la forme d'une ou plusieurs broches (144) dans la partie transmuqueuse (122), ou
(iii) est inclus dans une cavité (134) dudit implant dentaire (118) ou élément de celui-ci, dans lequel ladite cavité (134) est reliée à l'environnement par l'intermédiaire d'au moins une ouverture (136) comprise dans ladite partie transmuqueuse (122) ; ou
(iv) est inclus dans un moyen amovible pouvant être monté sur ladite partie transmuqueuse (122).

13. Utilisation d'un biocide pour fabriquer un implant dentaire (118) ou un élément de celui-ci, ledit implant dentaire (118) étant un implant dentaire selon l'une quelconque des revendications 1 à 7.
